# EUROPEAN PATENT SPECIFICATION

(11) **EP 0 710 653 B1**
(45) Date of publication and mention of the grant of the patent: **03.09.1997**
(21) Application number: 95307812.8
(22) Date of filing: 01.11.1995
(51) Int. Cl.: C07D 249/12, A61K 31/495

(54) **Salts of nefazodone having improved dissolution rates**
Nefazodon-Salze mit verbesserter Auflösungsgeschwindigkeit
Sels de la néfazodone ayant une vitesse de dissolution améliorée

(30) Priority: 02.11.1994 US 333390
(43) Date of publication of application: 08.05.1996
(73) Proprietor: Bristol-Myers Squibb Company, New York, N.Y. 10154 (US)
(72) Inventor: Joshi, Hemant N., Dayton, New Jersey (US); Wilson, Terry D., Albany, New York (US); Patel, Jatin M., Voorhees, New Jersey (US)
(74) Representative: Jones, Alan John

(56) References cited:
- WO-A-94/18978
- FR-A- 2 551 439

## Description

The invention deals with salts of the psychotropic medicament nefazodone, which have higher dissolution rates and faster release rates in the intestinal tract. Because of their greater intestinal absorption, these salts are usable in sustained- or extended-release formulations.

Nefazodone is an antidepressant agent. Its hydrochloride salt has structure (A):

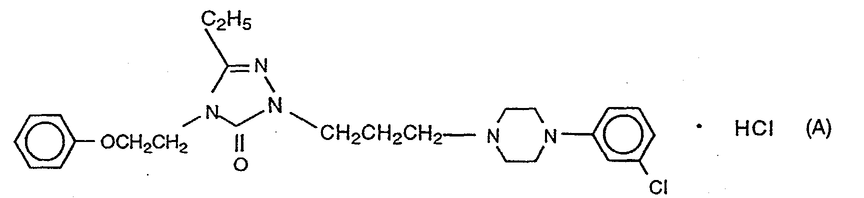

The preparation of nefazodone and its hydrochloride salt, nefazodone HCl, are described in U.S. 4,338,317 to Temple et al, in Examples 1 and 2, respectively.

Nefazodone has a pKa of 6.4 and low aqueous solubility at neutral pH. Its poor bioavailability in an extended release formulation in fasting patients is attributable to its low solubility at pH's of 5 to 7, i.e., those found in the human intestinal tract.

The use of nefazodone salts which dissolve faster at intestinal pH's in oral formulations is one way to address this problem. These salts are useful in oral extended release preparations which are believed to have good bioavailability.

The process of identifying suitable salts is not straight forward. That is because it is difficult to predict the solubilities and other physicochemical properties of salts of complex molecules, such as nefazodone.

The invention relates to certain salts of nefazodone which exhibit higher dissolution rates and faster release rates at basic pH's, i.e., at pH's found in the intestinal tract, when compared to other salts. These salts--i.e., the L-malate, mesylate and L-tartrate salts of nefazodone-dissolve faster at relatively high pH's. Accordingly, they are expected to yield oral formulations, which may be more bioavailable over extended periods than ones containing other nefazodone salts, e.g., the hydrochloride.

### BRIEF DESCRIPTION OF THE DRAWINGS

Figure 1 shows the dissolution profiles (i.e., intrinsic dissolution rate determinations) of the salts indicated, plotted as mg of cumulative equivalent nefazodone base dissolved vs. time, in minutes, in tri(hydroxymethyl)aminomethane (THAM) buffer(0.5 M, pH 7.7) (intrinsic dissolution rate determination).

Figure 2 depicts the level of nefazodone base released, in mg/mL, plotted against minutes when powders of these salts were dissolved in 0.125 M THAM, pH 7.5 (equilibrium solubility determination).

Figure 3 is a plot of % nefazodone salt released into simulated gastric fluid (SGF) from tablets vs. time (pH 1.2). The salts used are listed.

Figure 4 shows the dissolution profile of the salts listed, shown as % nefazodone released from tablets into simulated intestinal fluid (SIF, pH 7.5) vs. time (tablet formulation).

Figure 5 illustrates the % of nefazodone salt released from tablets in 0.5 M THAM buffer at pH 7.5 vs. time in hours (tablet formulation).

The invention deals with salts of nefazodone which exhibit advantageous physicochemical properties, e.g., better dissolution rates, in environments having near neutral pH's found in the human intestinal tract. The pH in the duodenum is about 6.5 and the pH is higher in lower sections of the intestine. See Drug Formulation by I. Racz, John Wiley & Sons of, NY, (1989).

The salts of the invention and their preparation, as well as compositions and methods employing them, are described.

The salts of the invention are prepared by contacting commercially available L-malic, L-tartaric and methane sulfonic (i. e., mesylic) acids with nefazodone base under suitable conditions. One or more of the resultant salt(s) is then employed in compositions and methods for delivering nefazodone to subjects.

Unless stated otherwise, all percentages recited herein are weight percents, based on total composition weight. All references recited are hereby incorporated by reference.

### EXAMPLES

The following examples set forth the preparation of nefazodone base and the derivation of the salts of the invention therefrom.

Example 1 describes the preparation of nefazodone base and the salts discussed herein. Example 2 sets out intrinsic dissolution and release studies conducted using pellets and tablet formulations, respectively.

### Example 1: Preparation of Nefazodone Base

Nefazodone HCl was prepared in accordance with Example 2 of U.S. 4,338,317. Nefazodone HCl was dissolved in water by gentle heating (40-45°C). An equimolar quantity of sodium hydroxide solution (0.1 N) was added with constant stirring so as to neutralize the hydrochloric acid from nefazodone HCl. Ice was added to bring down the temperature of the solution. Nefazodone base precipitated quickly as a sticky mass. The supernatant layer was decanted and nefazodone base was then washed with a large quantity of water (1 liter, two to three times). This was performed to remove sodium chloride generated in the neutralization of hydrochloric acid. The washings were tested with silver nitrate for trace amounts of chloride ions. The filtered base was washed with water until no chloride ions were detected in the washings. Nefazodone base was then dissolved and crystallized from isopropyl alcohol (IPA).

The potency of the nefazodone base was determined by HPLC assay using nefazodone HCI as the reference compound. The description of the HPLC assay is given below (Method 1).

The average melting point of nefazodone base from 7 batches was 82.4 ± 1.1°C, with a heat of fusion value of 19.5 ± 1.2 cal/g, as determined by differential scanning calorimetry (DSC).

### Preparation of Salts

Nefazodone base (1 gram) was dissolved in 6-8 mL of isopropyl alcohol (IPA) using gentle heating. Various acids (1.05 mole equivalent) were dissolved in 2 mL of IPA. The two solutions were mixed and stirred thoroughly using a magnetic stirrer. In most cases, the salt precipitated immediately and was filtered and washed with IPA. Any excess acid was believed to remain in solution. Any excess acid absorbed on the filtered salt was removed during washings with IPA.

In some cases, salts did not precipitate readily and IPA was evaporated at room temperature to recover the salt. In these cases, no attempt was made to remove the excess acid from the salt of nefazodone.

All the salts were dried in the oven at 50°C overnight. Melting points and the heats of fusion were determined by DSC.

A general scheme procedure for making nefazodone salts is set forth below.

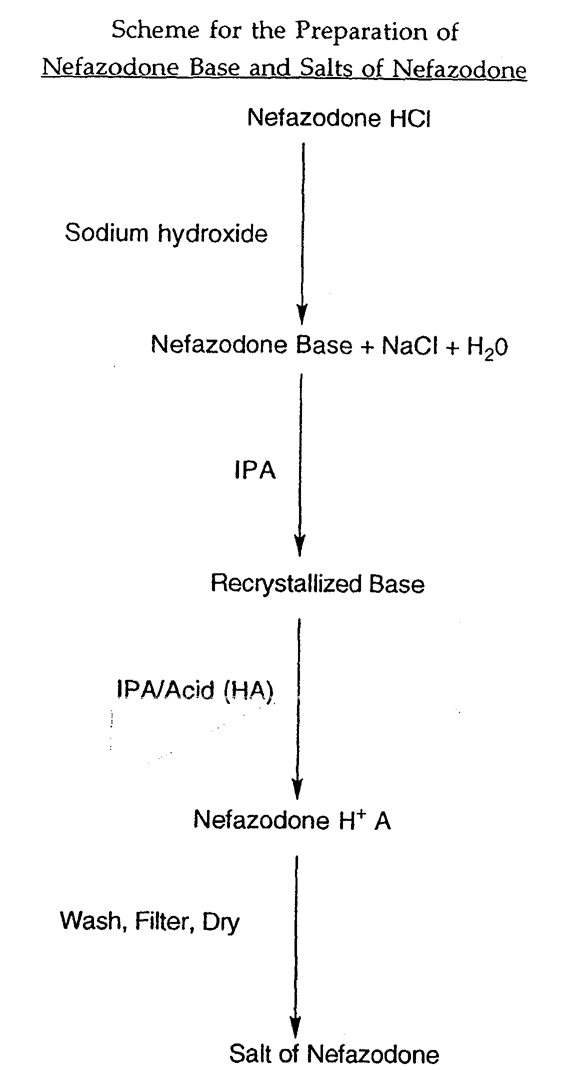

L-malic acid is also known as hydroxybutanedioic acid. It is the naturally occurring isomer of malic acid, found in apples and other plants. It is described as "apple acid" under Compound #5529 (p. 814) of The Merck Index, 10th edition (1983). It is sold by Fluka Chemika.

L-tartaric acid, L-2,3-dihydroxybutanedioic acid or natural tartaric acid, is a fruit acid widely found in nature. It is Compound #8945 (p. 1303) in The Merck Index, 10th edition(1983). It can be purchased from Sigma Chemicals.

Methane sulfonic acid, or mesylic acid, is a staple reagent prepared from sulfur trioxide and methane or via the oxidation of dimethyl disulfide. It is compound #5813 (p. 853) in The Merck Index, 10th edition (1983). Sigma Chemicals is a supplier.

The procedure adopted to make various salts, such as the L-malate, was similar to that used in the preparation of the hydrochloride salt of nefazodone. IPA was warmed to 50-60°C to dissolve nefazodone base, to which the solution of acid in IPA was added and upon cooling the salt precipitated. With some acids, such as citric acid, the salts formed a white emulsion, which solidified upon cooling in the refrigerator for 48-72 hours. However, it was not possible to filter the citrate salt, as it melted at room temperature.

Some salts did not precipitate even after cooling in the refrigerator. In such instances (i.e., acetate, L-lactate and ethanesulfonate salts), the IPA solution of the salt was evaporated to yield a viscous solution which produced small quantities of solid salts.

During the synthesis of the adipate and phosphate salts of nefazodone, dark brown liquids were formed. They did not crystallize even after evaporation of IPA. Further efforts to make these two salts were abandoned.

The DL-lactate salt of nefazodone did not crystallize during the synthesis and was not studied further. The acetate salt obtained was light brown in color, while other salts were white.

Salts obtained in a sizable quantity were recrystallized from IPA containing 2% water. The solubilities of salts were found to be much higher in IPA with 2% water compared to that in pure IPA. The volumes of IPA containing 2% water needed to dissolve the hydrochloride and malate salts of nefazodone were 23 mL/g and 20 mL/g, respectively. The L-tartrate salt could not be dissolved in IPA with 2% water over a concentration of 0.036 g/mL and therefore, the recrystallization of this salt was not carried out.

Pure IPA was used to recrystallize the mesylate, i.e., methane sulfonate salt of nefazodone. Traces of water in IPA produced a material with an additional endotherm at 110°C in the DSC analysis for the mesylate (methane sulfonate) salt. Hot stage microscopic analysis of the mesylate showed outgassing at this temperature, suggesting the presence of a hydrate of nefazodone mesylate.

Recrystallization of nefazodone mesylate was attempted with various solvents. The mesylate recrystallized from polar solvents such as ethanol, methanol and acetonitrile. It showed a melting endotherm at 110°C by DSC and also outgassing at the same temperature in hot stage microscopy. Such solvents may contain a small percent of water as an impurity, which would cause the formation of an apparent nefazodone mesylate hydrate melting at 110°C.

Table 1 shows melting point and heat of fusion data for various nefazodone salts.

**Table 1**

| Melting Points and Heats of Fusion (n=2) of Various Salts of Nefazodone | | | |
|---|---|---|---|
| Salt | | Melting Point, °C | Heat of Fusion, Cal/g |
| Nefazodone base | | 82.4± 1.1# | 19.5± 1.2# |
| Hydrochloride* | Lot I | 186 | 30.1 |
| | Lot II | 180 | 28.9 |
| Malate | D | 105 | 21.6 |
| | DL | 108 | 21.8 |
| | L | 116 | 22.7 |
| Tartrate | D | 93 | 16.7 |
| | DL | 84 | 14.6 |
| | L | 94 | 15.6 |
| Mesylate | | 158*** | 27.4*** |
| Tosylate | | 159 | 22.9 |
| Maleate | | 150 | 22.9 |
| Succinate | | 98 | 25.9 |
| Acetate | | 76 | 17.0 |

| | | | |
|---|---|---|---|
| *Nefazodone HCl: Lot I- material obtained from Humacao Lot II - material synthesized in the basic pharmaceutical laboratory, Evansville, Indiana. | | | |
| *** n = 4 | | | |
| # n = 7 n = number of replicates | | | |

Melting points and heat of fusion values were obtained from differential scanning calorimetry (DSC).

Table 2 shows the results of thermogravimetric (TGA) analysis and the observed and theoretical potency values for the selected salts.

TGA of the hydrochloride, L-tartrate, L-malate and mesylate salts of nefazodone were performed in the temperature range 40-180°C at the heating rate of 10°C/minute (n = 1).

Potencies of the hydrochloride, L-tartrate, L-malate and mesylate salts of nefazodone were determined by HPLC analysis using nefazodone base as the standard. The details of HPLC are summarized below (Method 1). In the calculations, the potency values were corrected for moisture (or solvent residue) as judged by the percent weight loss observed in the TGA experiments.

**Table 2**

| Percent Weight Loss in Thermogravimetric Analysis (TGA) and Percent Potency of Salts of Nefazodone | | | | |
|---|---|---|---|---|
| Salt | % Weight Loss in TGA | Moles Water Per Mole Salt | Theoretical Potency, % | Observed Potency %* |
| Hydrochloride | 0.0 | 0.0 | 92.8 | 92.5±2.1 |
| L-malate | 1.1 | 0.4 | 77.8 | 77.4±1.1 |
| Mesylate | 2.3 | 0.7 | 83.0 | 82.6±0.5 |
| L-tartrate | 4.3 | 1.5 | 75.8 | 72.8±1.4 |

| | | | | |
|---|---|---|---|---|
| * Mean value of two samples injected four times each. Values were also corrected for the weight loss observed in TGA experiments. | | | | |

The L-tartrate salt shows 4.3% weight loss (w/w). If this loss of weight is due to water, nefazodone L-tartrate contains 1.5 moles of water (per mole of salt). Nefazodone L-malate and mesylate showed 1.1% and 2.3% weight loss respectively (corresponding to 0.4 and 0.7 moles water per mole of salt), whereas nefazodone hydrochloride did not show any weight loss.

In the determination of potency of the salts, nefazodone base was used as the reference material. Thus, depending on the molecular weight of each salt, the theoretical potency values were corrected for the percent weight loss in the TGA analysis. The observed and theoretical values were comparable, indicating good purity of these salts.

### Example 2: Dissolution and Release Studies

Preparation of Pellets. Pellets of the salts (approx. 50 to 100 mg.) were made on a Carver Press, Model C. Pressure to make nefazodone HCI pellets was about 4,000 psi, while pressure of less than 50 psi was needed to make the pellets of other salts of nefazodone. Attempts to apply higher pressure resulted in the breaking of the pellets. Pellets were mounted in white wax on a glass slide. Precaution was taken to keep open the top surface of the pellet, so that the dissolution of drug would occur only from the top surface (approximate diameter of 0.25 inch).

The intrinsic dissolution experiments were performed with 75 mL water as the dissolution medium. The details of the dissolution experiments are summarized below:

Kettle used: Double-walled beaker containing 100 mL medium, round bottom.

Temperature: 37°C.

Volume of sample withdrawn per interval: 1 mL.

Temperature of the solution in the beaker was maintained by means of a circulating waterbath. The medium was stirred with a USP II paddle using 100 rpm stirring rate (paddle depth = 1 inch below the surface of the medium). Samples were withdrawn through a 0.45 µm syringe filter and were analyzed without any further dilution or treatment by HPLC (Method 1).

### Intrinsic Dissolution Experiments in THAM

THAM buffer (0.5 M, pH 7.7) was prepared by dissolving tri(hydroxymethyl)aminomethane in water and adjusting the pH with concentrated hydrochloric acid. Dissolution of salts of nefazodone in pellet form was studied in this medium with the experimental procedures discussed above.

### Powder Dissolution Experiments in THAM

The experiments were conducted in 0.125 M THAM (pH 7.7) at 37°C using a USP II dissolution apparatus in the Oral Solids Laboratory. Powders (about 75-80 mg) were added to 900 mL dissolution medium stirred at 50 rpm and samples were withdrawn at time points up to 2 hours. The USP official method recommends paddles to be 1 inch from the bottom. In this experiment, paddles were 3/4 inch from the bottom. The paddle depth was altered to facilitate suspension of the powder during experiments. Samples were analyzed using the HPLC assay.

### Release Profiles from the Tablet Formulation

The preparation and characterization of tablets of salts of nefazodone was performed as described below:
Ingredients used in the tablets of salts of nefazodone are listed in Table 3.

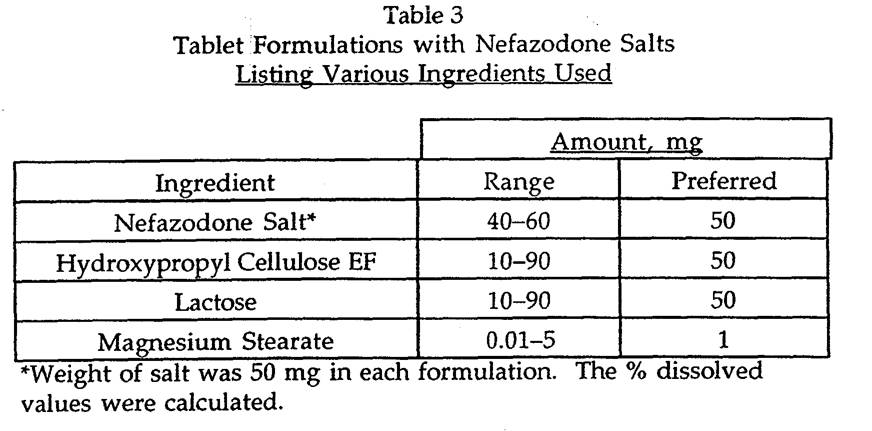

Drug substance, hydroxypropyl cellulose EF, and lactose were mixed for 10-15 minutes. Magnesium stearate was added to this mixture, mixing it for five more minutes. Tablets were compressed using a Manesty machine (Model-Manesty B3B). The tablet hardness was approximately 7 SCU.

Dissolution studies were performed with a USP II apparatus in simulated gastric fluid (USP, pH 1.2), simulated intestinal fluid (USP, pH 7.5) and 0.5 M THAM (pH 7.7). The details of conditions for the dissolution experiments are as follows:
Volume of the medium withdrawn at each interval: 900 mL.
Sample volume withdrawn at each interval: 0.7 mL.
Temperature: 37°C.
Stirring speed: 50 rpm.

Samples were filtered during withdrawal and chromatographed without further dilution or treatment. Samples were analyzed using the HPLC conditions listed in Method 2, below.

### HPLC Conditions

### Method 1

Column: Novapak, C18, 7.5 cm
Mobile Phase: ACN:water:diethyl amine (600:400:0.5 v/v)
Wavelength: 254 nm
Flow rate: 1.25 mL/min
Retention time: 2.2 minutes
Injection volume: 20 µL

### Method 2

Column: µ Bondapak, C18, 15 cm.
Mobile Phase: Methanol:0.01 M Ammonium Phosphate
buffer, pH 6.0 (85:15, v/v)
Wavelength: 254 nm
Flow rate: 1.0 mL/min
Retention time: 2.5 minutes

When SCF was used as the dissolution medium, Method 2 was slightly modified. Methanol:buffer ratio in the mobile phase was 75:25 and the flow rate was 1.2 mL/min.

Pressures used to make pellets of the hydrochloride and other salts of nefazodone were 4000 psi and less than 50 psi, respectively.

Table 4 shows, for various salts, the intrinsic dissolution rates in water at 37°C and the final pH values of the solutions. The table lists the values for D-, L-, and DL-stereoisomers of malate and tartrate salts.

**Table 4**

| Intrinsic Dissolution Rates (n = 2) of and Final pH Values of Various Salts of Nefazodone in Water at 37 C.° | | | |
|---|---|---|---|
| Salt | | Dissolution Rate (mg/min/cm²) | Final pH* |
| Nefazodone base | | 0.002** | 7.2 |
| Hydrochloride | Lot - I | 0.21 | 5.3 |
| | Lot - II | 0.93 | 4.4 |
| Malate | D | 1.15 | 4.2 |
| | DL | 0.98 | 4.3 |
| | L | 1.36 | 4.2 |
| Tartrate | D | 1.13 | 3.7 |
| | DL | 1.2 | 3.7 |
| | L | 0.97 | 3.8 |
| Mesylate | | 2.7 | 4.0 |
| Tosylate | | 0.1 | 4.4 |
| Maleate | | 0.30 | 4.8 |
| Succinate | | 0.16 | 5.0 |
| Acetate | | 0.03 | 5.4 |

| | | | |
|---|---|---|---|
| * Final pH of medium after the dissolution experiment. | | | |
| ** n=1 | | | |

The intrinsic dissolution rate for nefazodone base in water (0.002 mg/min/cm², pH 7.2) was low compared to the salts. The intrinsic dissolution rate of the hydrochloride salt (Lot II) was 4.4 times the rate of the hydrochloride salt (Lot I). The final pH of the solutions for the Lot II and Lot I materials were 4.4 and 5.3, respectively. In further discussion, nefazodone HCl from Lot I will be called "nefazodone hydrochloride".

The intrinsic dissolution rate of L-malate was slightly higher compared to the other two stereoisomers and the value was 6.5 times higher than that of nefazodone HCl.

Nefazodone L-tartrate showed faster dissolution (4 times) than nefazodone hydrochloride in water. D- and DL-tartrates showed slightly higher dissolution rates compared to the L-tartrate. L-tartrate was selected for further studies as it is available at a lower cost.

The mesylate salt of nefazodone (melting point 159°C) had the highest intrinsic dissolution rate, which was 13 times that for the hydrochloride salt. The final pH was also lower (pH 4.0), but not the lowest of the salts tested.

Other salts showed intrinsic dissolution rate values either comparable to or less than the hydrochloride salt.

No correlation was observed between the melting points and the intrinsic dissolution rates. Based on the higher intrinsic dissolution rates, L-malate, L-tartrate and mesylate salts were selected for further study.

In the dissolution experiments of salts of nefazodone in THAM buffer (0.5 M, pH 7.7), HPLC analysis showed 2 mg of hydrochloride salt of nefazodone dissolved in 75 mL from 250 mg pellets (discs). However, pellets of L-malate, L-tartrate and mesylate salts of nefazodone eroded significantly (about 30-40%). The pellets of the hydrochloride salt of nefazodone did not erode. Powders from the pellets suspended in a fine form and therefore, filtration of the solutions was essential (Acrodisc, 0.45 µm syringe filters).

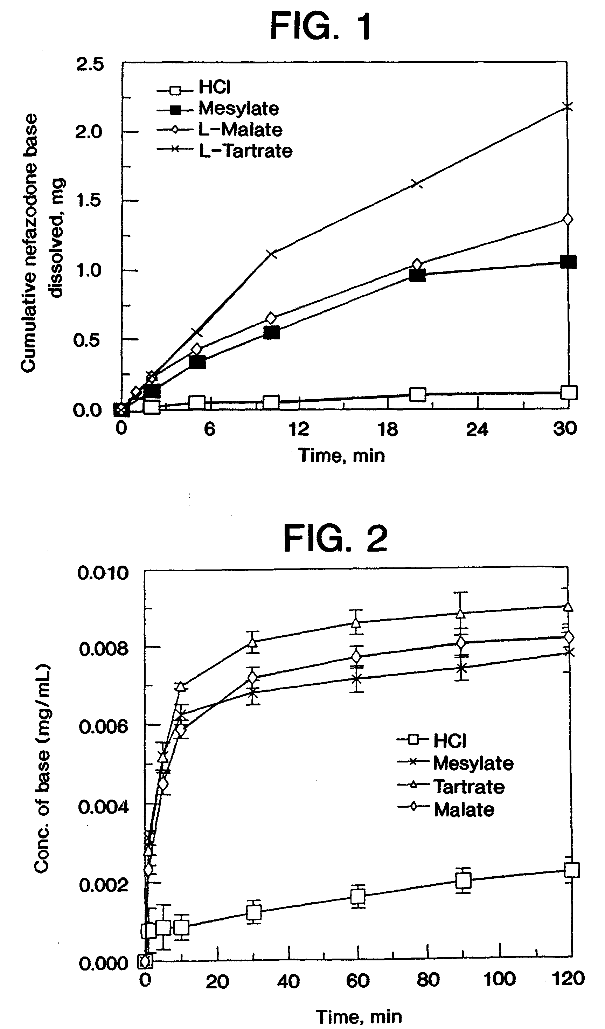

Table 5 and Figure 1 describe the results of intrinsic dissolution rate studies in THAM.

**Table 5**

| Initial Dissolution Rates of Selected Salts of Nefazodone in 0.5 M THAM buffer, pH 7.7. | | | |
|---|---|---|---|
| Salt | Final pH* | Initial Dissolution Rate** | r^2 |
| Hydrochloride | 7.4 | 0.003 | 0.631 |
| Mesylate | 7.3 | 0.040 | 0.986 |
| L-Tartrate | 7.3 | 0.082 | 0.999 |
| L-Malate | 7.3 | 0.046 | 0.963 |
| r^2 is the square of the coefficient of correlation | | | |

| | | | |
|---|---|---|---|
| * pH of the medium 30 minutes after dissolution experiment | | | |
| **The dissolution rate was determined by data regression between 0 to 10 minutes. The units are mg/min/cm². | | | |

Because of erosion of pellets, the surface area varied over time and, therefore, the rates of dissolution could be termed as the "intrinsic" only at initial times. However, as expected, the initial dissolution rates in THAM buffer were lower, compared to the rates in water. The rates were calculated from the regression analysis of data points from 0 to 10 minutes. The initial dissolution rate of the mesylate and L-malate salts were nearly 15 times that found for the hydrochloride salt. The initial dissolution rate of L-tartrate salt of nefazodone was 27 times that of the hydrochloride salt.

Figure 1 shows the dissolution profiles of these salts in 0.5 M THAM (initial buffer pH = 7.7). It is evident that the three salts showed significantly higher dissolution rates at pH 7.3 compared to the hydrochloride salt. The higher dissolution rates could be partially due to the suspension of the drug in the medium from the pellets for these salts compared to the hydrochloride salt. The shapes of the dissolution profile curves do not demonstrate the eroding nature of some of the pellets, suggesting a near-saturation of the dissolution media.

Figure 2 shows the dissolution of powders of various salts of nefazodone in 0.125 M THAM buffer, pH 7.5. The final pH values of the dissolution media after 2 hours were between 7.2 and 7.3. Salts of nefazodone were used as standards and the concentrations of salts obtained at different time points were converted to the amount of base dissolved (n = 3). It is clear from Figure 2 that L-malate, L-tartrate and mesylate salts showed significantly higher initial dissolution rates than the hydrochloride salt. The L-tartrate salt of nefazodone showed the fastest dissolution profile. As the drug will be absorbed continuously from the intestine, the higher dissolution rate could increase bioavailability.

### Release Profiles From Tablet Formulation

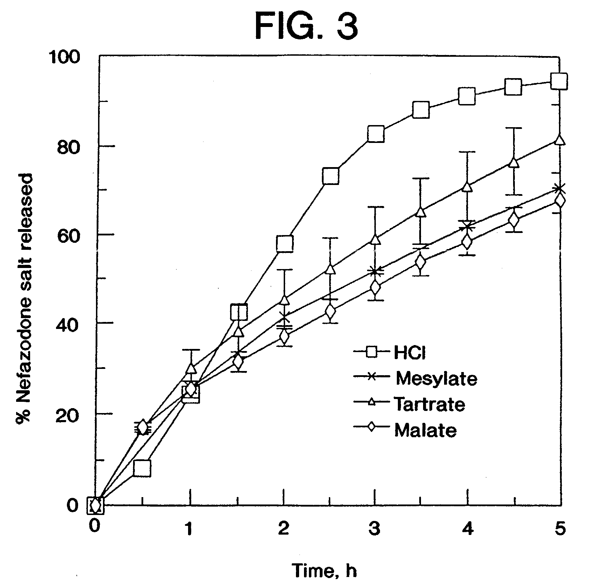

Simulated Gastric Fluid (SGF). Figure 3 shows the dissolution profiles of various salts from tablets in SGF (pH 1.2) at 37°C (n = 3) using a USP II apparatus. The salts of nefazodone (mesylate, L-tartrate and L-malate) showed somewhat higher initial dissolution rates (up to 1 hour). However, the hydrochloride salt of nefazodone showed the overall highest dissolution in 5 hours. The t_{50%} values of the other three salts were lower compared to the hydrochloride salt (Figure 3). t_{50%} means time required to release 50% drug from a tablet formulation.

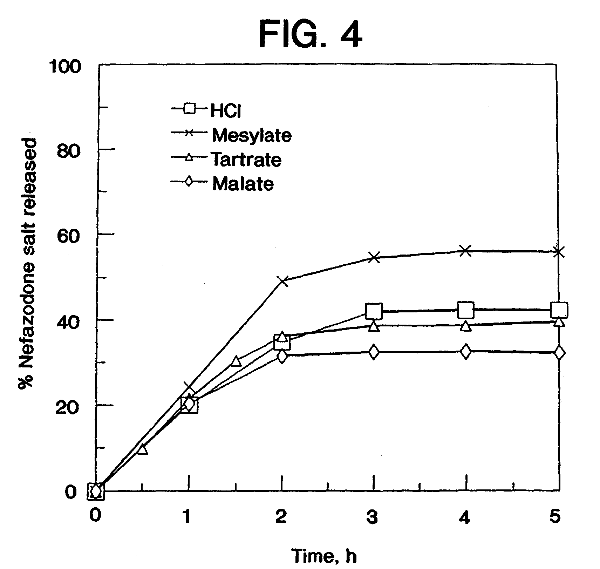

Simulated Intestinal Fluid (SIF). The dissolution profiles of various salts from the tablets in SIF (pH 7.5) is depicted in Figure 4. The mesylate salt showed the fastest dissolution profile while the profiles for the other three salts were similar. The salts studied may have formed a low solubility phosphate salts in the simulated intestinal fluid and, therefore, showed similar dissolution profiles.

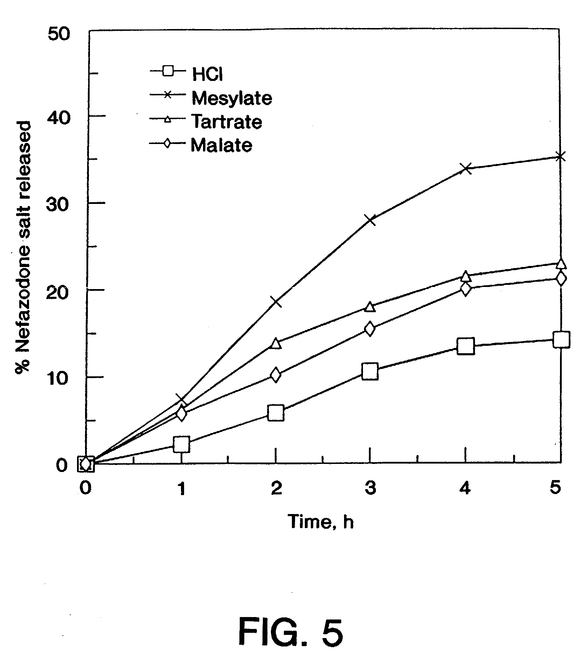

THAM Buffer, pH 7.5. The hydrochloride salt of nefazodone showed the slowest dissolution from tablets in 0.5 M THAM buffer, pH 7.5 (Figure 5). The mesylate salt of nefazodone showed fastest dissolution at the neutral pH with a final concentration of 0.01 mg/mL. As stated earlier, in SIF, all the salts might have converted to the phosphate salt yielding these dissolution profiles. THAM buffer could be better than SIF as a dissolution medium, only if it does not interact with nefazodone chemically, i.e., by forming a salt or an addition product.

The invention deals with the administration of nefazodone salts via orally-ingested dosage forms. Thus tablets, capsules, caplets, lozenges, powders, suspensions, syrups and the like are suitable forms. The use of tablets is preferred.

Suitable oral formulations contain one or more of the nefazodone salts in tablets having hardnesses of about 5 to about 7 SCU, with 7 SCU most preferred.

The oral compositions may contain a variety of conventional pharmaceutically acceptable excipients in effective amounts suitable for their respective functions. Thus, suitable amounts of conventional additives, such as the following, are useful: polymeric matrixes (e.g., chitosan, hydroxyalkylcelluloses), auxiliary binding agents (e.g., syrup, acacia, gelatin, sorbitol, tragacanth, or polyvinyl pyrrolidone), fillers (e.g., lactose, sugar, maize-starch, calcium phosphate, sorbitol or glycine), lubricants (e.g., magnesium stearate, cellulose, talc, polyethyleneglycol or silicas), disintegrants (e.g., starch), wetting agents (e.g., sodium lauryl sulfate), colorants (e.g., iron oxides), etc. Mixtures can be used.

Generally, the compositions of the invention will contain from about 20 to about 40 wt% of one or more nefazodone salts as the active pharmaceutical agents and from about 80 to about 60 wt% of pharmaceutically acceptable carrier(s), such as release extending agents and other excipients.

Effective doses of nefazodone salts of about 0.01 to about 40 mg/kg body weight are contemplated. For certain disorders, about 15 to about 90 mg/dose, preferably about 30 to about 60 mg/dose, are recommended. Generally, about 200 to about 300 mg/day are given. Dosage levels may vary according to the medical needs of the subject (e.g., the human patient or other host). In any event, sound medical judgment, such as that exercised by a licensed physician, should be used in determining optimal dosages.

The compositions and dosage forms discussed herein are designed to deliver an effective antidepressant amount of one or more nefazodone salt(s) to a mammal, preferably a human patient.

Reasonable variations, such as those which would occur to a skilled artisan, can be made herein without departing from the scope of the invention.

## Claims

1. A nefazodone salt having an aqueous intrinsic dissolution rate of about 0.04 to about 0.08 mg/min/cm², wherein the salt is selected from the group consisting of: the L-malic, L-tartric and methanesulfonate salts of nefazodone.

2. Nefazodone L-malate.

3. Nefazodone L-tartrate.

4. Nefazodone methane sulfonate.

5. A composition useful for orally administering nefazodone which contains:
(a) an effective antidepressant amount of at least one compound selected from the group consisting of the L-malate, L-tartrate and methane sulfonate salts of nefazodone, and
(b) a suitable amount of a pharmaceutically acceptable carrier.

6. The composition of claim 5 wherein the salt is the L-malate salt.

7. The composition of claim 5 wherein the salt is the L-tartrate salt.

8. The composition of claim 5 wherein the salt is the methane sulfonate salt.

9. An oral dosage form made from the composition of claim 5.

10. An oral dosage form made from the composition of claim 6.

11. An oral dosage form made from the composition of claim 7.

12. An oral dosage form made from the composition of claim 8.

13. A nefazodone salt selected from the L-malate, L-tartrate and methane sulfonate salts thereof, and pharmaceutical compositions comprising said salt, for use as a psychotropic medicament.

14. Salt of claim 13 wherein the salt is the L-malate salt.

15. Salt of claim 13 wherein the salt is the L-tartrate salt.

16. Salt of claim 13 wherein the salt is the methane sulfonate salt.

17. Use of a salt according to any one of claims 1 to 4, or a composition comprising said salt, for the manufacture of a psychotropic medicament.

## Revendications

1. Sel de néfazodone ayant une vitesse de dissolution intrinsèque dans l'eau d'environ 0,04 à environ 0,08 mg/mn/cm², où le sel est sélectionné dans le groupe consistant en sels L-malique, L-tartrique et méthanesulfonate de néfazodone.

2. L-malate de néfazodone.

3. L-tartrate de néfazodone.

4. Méthane sulfonate de néfazodone.

5. Composition utile pour l'administration orale de néfazodone qui contient :
(a) une quantité antidépressive efficace d'au moins un composé sélectionné dans le groupe consistant en sels de L-malate, L-tartrate et méthane sulfonate de néfazodone, et
(b) une quantité appropriée d'un support pharmaceutiquement acceptable.

6. Composition de la revendication 5 où le sel est le sel de L-malate.

7. Composition de la revendication 5 où le sel est le sel de L-tartrate.

8. Composition de la revendication 5 où le sel est le sel de méthane sulfonate.

9. Forme de dosage oral faite de la composition de la revendication 5.

10. Forme de dosage oral faite de la composition de la revendication 6.

11. Forme de dosage oral faite de la composition de la revendication 7.

12. Forme de dosage oral faite de la composition de la revendication 8.

13. Sel de néfazodone sélectionné parmi les sels de L-malate, L-tartrate et méthane sulfonate, et compositions pharmaceutiques comprenant le sel, à utiliser comme médicament psychotrope.

14. Sel de la revendication 13 où le sel est le sel de L-malate.

15. Sel de la revendication 13 où le sel est le sel de L-tartrate.

16. Sel de la revendication 13 où le sel est le sel de méthane sulfonate.

17. Utilisation d'un sel selon l'une quelconque des revendications 1 à 4, ou une composition comprenant ledit sel, pour la fabrication d'un médicament psychotrope.

## Patentansprüche

1. Nefazodonsalz mit einer intrinsischen Lösungsgeschwindigkeit in Wasser von etwa 0,04 bis etwa 0,08 mg/Min./cm², wobei das Salz ausgewählt ist unter Nefazodon-L-Äpfelsäuresalz, Nefazodon-L-Weinsäuresalz und Nefazodon-Methansulfonsäuresalz.

2. Nefazodon-L-Äpfelsäuresalz.

3. Nefazodon-L-Weinsäuresalz.

4. Nefazodon-Methansulfonsäuresalz.

5. Zusammensetzung, die zur oralen Verabreichung von Nefazodon geeignet ist, und enthält:
(a) eine antidepressiv wirksame Menge von mindestens einer Verbindung ausgewählt unter Nefazodon-L-Äpfelsäuresalz, Nefazodon-L-Weinsäuresalz und Nefazodon-Methansulfonsäuresalz, und
(b) eine geeignete Menge eines pharmazeutisch verträglichen Trägers.

6. Zusammensetzung nach Anspruch 5, worin das Salz das L-Äpfelsäuresalz ist.

7. Zusammensetzung nach Anspruch 5, worin das Salz das L-Weinsäuresalz ist.

8. Zusammensetzung nach Anspruch 5, worin das Salz das Methansulfonsäuresalz ist.

9. Orale Darreichungsform, hergestellt aus der Zusammensetzung nach Anspruch 5.

10. Orale Darreichungsform, hergestellt aus der Zusammensetzung nach Anspruch 6.

11. Orale Darreichungsform, hergestellt aus der Zusammensetzung nach Anspruch 7.

12. Orale Darreichungsform, hergestellt aus der Zusammensetzung nach Anspruch 8.

13. Nefazodonsalz ausgewählt unter dem L-Äpfelsäuresalz, dem L-Weinsäuresalz und dem Methansulfonsäuresalz davon, und pharmazeutische Zusammensetzungen, die das genannte Salz umfassen, zur Verwendung als psychotropes Medikament.

14. Salz nach Anspruch 13, worin das Salz L-Äpfelsäuresalz ist.

15. Salz nach Anspruch 13, worin das Salz das L-Weinsäuresalz ist.

16. Salz nach Anspruch 13, worin das Salz das Methansulfonsäuresalz ist.

17. Verwendung eines Salzes nach einem der Ansprüche 1 bis 4 und einer Zusammensetzung, die das genannte Salz umfasst, zur Herstellung eines psychotropen Medikaments.
